# DEMANDE DE BREVET EUROPEEN

(11) **EP 2 990 472 A1**
(43) Date de publication de la demande: **02.03.2016**
(21) Numéro de dépôt: 15290221.9
(22) Date de dépôt: 24.08.2015
(51) Int. Cl.: C11D 9/12, C11D 13/10, A61Q 19/10, A61K 8/92, A61K 8/97

(54) **COMPOSITION DÉTERGENTE ET PROCÉDÉ DE FABRICATION DE LADITE COMPOSITION DÉTERGENTE**

(30) Priorité: 29.08.2014 FR 1401945
(71) Demandeur: Scheuer, Guy, 67120 Molsheim (FR)
(72) Inventeur: Scheuer, Guy, 67120 Molsheim (FR)

(57) **Abrégé**

La présente invention concerne une composition détergente comportant :
c) entre 60% et 80% en grammes d'un mélange contenant:
- entre 50% et 65% en grammes de bicarbonate de sodium
- 7% en grammes de carbonate de sodium ou entre 8% et 10% en grammes de carbonate de sodium
- entre 2% et 5% en grammes de bicarbonate d'ammonium, et

d) entre 20% et 40% en grammes d'une solution de savon noir à 15%.

## Description

La présente invention concerne le domaine des produits d'entretien et vise à proposer une composition détergente polyvalente, biodégradable et non agressive pour l'utilisateur, de sorte à permettre, le cas échéant, une utilisation en tant que produit d'hygiène corporelle.

De nombreuses compositions détergentes classiques, disponibles sur le marché à l'heure actuelle, sont généralement spécifiquement étudiées et formulées pour répondre de manière efficace à une application particulière.

Ainsi, pour un résultat impeccable, il est généralement nécessaire de recourir à un produit différent pour chaque tâche ménagère à accomplir. Par exemple les détergents classiquement conçus pour une utilisation en lave-vaisselle ne conviennent pas au lavage manuel de la vaisselle et encore moins en tant que dentifrice.

De même, un produit à vitres ne permet pas par exemple de se laver les mains et n'est pas davantage adapté par exemple à l'entretien d'un sol en bois.

D'autre part, le présent demandeur est particulièrement sensible à l'impact négatif que peuvent avoir certaines compositions détergentes sur l'environnement naturel et par conséquent sur la santé des hommes et des animaux, suite à leur rejet dans les eaux usées.
Un aspect prépondérant de la présente invention est donc de proposer une composition détergente polyvalente qui soit en outre totalement biodégradable et donc inoffensive pour l'ensemble des plantes et des êtres vivants.

A cet effet, la présente invention se rapporte à une composition détergente comportant :
a) entre 60% et 80% en grammes d'un mélange contenant:
   - entre 50% et 65% en grammes de bicarbonate de sodium
   - 7% en grammes de carbonate de sodium ou entre 8% et 10% en grammes de carbonate de sodium
   - entre 2% et 5% en grammes de bicarbonate d'ammonium, et
b) entre 20% et 40% en grammes d'une solution de savon noir à 15%

Conformément à une variante de réalisation préférentielle, la présente composition détergente comporte:
a) 70% en grammes d'un mélange contenant:
   - 60% en grammes de bicarbonate de sodium
   - 7% en grammes de carbonate de sodium
   - 3% en grammes de bicarbonate d'ammonium, et
b) 30% en grammes d'une solution de savon noir à 15%

Par ailleurs, la composition détergente selon l'invention peut également se caractériser en ce qu'elle comporte un additif, les proportions en grammes dudit mélange et de ladite solution étant conservées.

Cet additif peut être défini par un composant permettant d'améliorer l'aspect d'un article nettoyé au moyen de la composition détergente selon l'invention, tel que par exemple du silicate de soude garantissant le brillant ou un oxydant apte à éliminer toute trace de tanins, notamment de thé ou de café.

Selon une autre alternative, ledit additif peut être un parfum.

Selon une autre alternative, ledit additif peut être un colorant.

L'invention a également pour objet un procédé de fabrication de la composition détergente telle que définie ci-dessus, caractérisé en ce que l'on prépare d'abord ledit mélange puis on rajoute ladite solution et on malaxe jusqu'à l'obtention d'une pâte homogène.

Selon un mode de mise en oeuvre préférentiel du procédé, l'on prépare ledit mélange à partir de poudres de bicarbonate de sodium, de carbonate de sodium et de bicarbonate d'ammonium finement broyées.

L'exemple de réalisation figurant ci-après a pour objet d'illustrer la présente invention et ses avantages plus en détail.

### Exemple:

1 kilogramme d'une composition détergente comportant:
a) 70% en grammes d'un mélange contenant:
   - 60% en grammes de bicarbonate de sodium
   - 7% en grammes de carbonate de sodium
   - 3% en grammes de bicarbonate d'ammonium, et
b) 30% en grammes d'une solution de savon noir à 15% est obtenu par le procédé suivant:
   On mélange d'abord 600 grammes de bicarbonate de sodium, 70 grammes de carbonate de sodium et 30 grammes de bicarbonate d'ammonium, les trois composés étant utilisés sous forme de poudre finement broyée, présentant de préférence une granulométrie telle que lorsque la composition détergente est utilisée, elle présente un léger pouvoir abrasif.

Puis on rajoute audit mélange 300 grammes d'une solution de savon noir à 15% en grammes et on malaxe l'ensemble pour obtenir une pâte homogène.

Celle-ci présente une couleur blanchâtre, une odeur neutre, et un pH d'environ 8-9 pour 5 grammes dans 100 millilitres d'eau.

Cette composition peut être utilisée pure ou diluée. Elle se révèle particulièrement efficace en tant que produit de lavage pour lave-vaisselle. De plus, elle est agréable à manipuler et n'est ni irritante, ni corrosive. Par ailleurs, les eaux de rinçage obtenues sont parfaitement respectueuses de l'environnement.

Avantageusement, la composition détergente selon l'invention est polyvalente et peut être utilisée dans de nombreuses autres applications. Ainsi, elle a également été testée avec succès pour nettoyer de nombreux type de surfaces, vitrées, carrelées, boisées, en inox, etc., y compris particulièrement encrassées telles que notamment des jantes de voitures.

De même, son utilisation dans le cadre de l'hygiène corporelle a été testée. Ainsi, la présente composition s'est révélée adaptée par exemple au lavage des mains et au brossage des dents, notamment lorsqu'elle contient un additif tel qu'un parfum lui conférant un caractère proche de celui des produits classiques auquel le consommateur est habitué.

La présente invention n'est pas limitée à l'exemple de réalisation décrit mais s'étend à toute modification et variante évidentes pour un homme du métier tout en restant dans l'étendue de la protection définie dans les revendications annexées.

## Revendications

1. Composition détergente comportant :
a) entre 60% et 80% en grammes d'un mélange contenant:
- entre 50% et 65% en grammes de bicarbonate de sodium
- 7% en grammes de carbonate de sodium ou entre 8% et 10% en grammes de carbonate de sodium
- entre 2% et 5% en grammes de bicarbonate d'ammonium, et
b) entre 20% et 40% en grammes d'une solution de savon noir à 15%.

2. Composition détergente selon la revendication 1, **caractérisée en ce qu'**elle comporte
a) 70% en grammes d'un mélange contenant:
- 60% en grammes de bicarbonate de sodium
- 7% en grammes de carbonate de sodium
- 3% en grammes de bicarbonate d'ammonium, et
b) 30% en grammes d'une solution de savon noir à 15%.

3. Composition détergente selon la revendication 2, **caractérisé en ce qu'**elle comporte un additif, les proportions en grammes dudit mélange et de ladite solution étant conservées.

4. Composition détergente selon la revendication 3, **caractérisée en ce que** ledit additif est du silicate de soude.

5. Composition détergente selon la revendication 3, **caractérisée en ce que** ledit additif est un parfum.

6. Composition détergente selon la revendication 3, **caractérisé en ce que** ledit additif est un colorant.

7. Composition détergente selon la revendication 3, **caractérisée en ce que** ledit additif est un oxydant.

8. Procédé de fabrication de la composition détergente selon l'une quelconque des revendications 1 à 7, **caractérisé en ce que** l'on prépare d'abord ledit mélange puis on rajoute ladite solution et on malaxe jusqu'à l'obtention d'une pâte homogène.

9. Procédé de fabrication selon la revendication 8, **caractérisé en ce que** l'on prépare ledit mélange à partir de poudres de bicarbonate de sodium, de carbonate de sodium et de bicarbonate d'ammonium finement broyées.

10. Utilisation de la composition détergente selon l'une des revendications 1 à 7 en tant que produit de lavage pour lave-vaisselle.

11. Utilisation de la composition détergente selon l'une des revendications 1 à 7 en tant que produit d'hygiène corporelle.
